# EUROPEAN PATENT APPLICATION

(11) **EP 4 696 237 A1**
(43) Date of publication of application: **18.02.2026**
(21) Application number: 24896664.0
(22) Date of filing: 28.11.2024
(51) Int. Cl.: A61B 5/16

(54) **METHOD FOR TRAINING HUMAN-CENTERED INTELLIGENT PHYSIOLOGICAL STATE RECOGNITION MODEL, METHOD AND APPARATUS FOR PHYSIOLOGICAL STATE RECOGNITION, AND DEVICE**

(30) Priority: 30.11.2023 CN 202311628678; 26.12.2023 CN 202311810471
(71) Applicant: Kingfar International Inc., Beijing 100085 (CN)
(72) Inventor: ZHAO, Qichao, Beijing 100085 (CN); WANG, Qingju, Beijing 100085 (CN); YANG, Ran, Beijing 100085 (CN)
(74) Representative: Lewis Silkin LLP
(86) International application number: PCT/CN2024/135344
(87) International publication number: WO 2025/113579

(57) **Abstract**

Embodiments of the present disclosure provide a method for training a human-factors intelligent physiological state recognition model, a method and apparatus for physiological state recognition, and a device. The method includes: obtaining a first physiological signal, a first state label of the first physiological signal, a second physiological signal corresponding to the first physiological signal, and a second state label of the second physiological signal, in which the second physiological signal is a physiological signal generated based on the first physiological signal, the first physiological signal is a collected physiological signal, the first state label indicates a physiological state corresponding to the first physiological signal, and the second state label indicates a physiological state corresponding to the second physiological signal; and training a physiological state recognition model based on the first physiological signal, the second physiological signal, the first state label, and the second state label. The embodiments of the present disclosure can realize more accurate physiological state recognition of a subject.

## Description

### CROSS-REFERENCE TO RELATED APPLICATIONS

This application claims priorities to Chinese Patent Application No. 202311628678.X, titled "METHOD FOR TRAINING HUMAN-FACTORS INTELLIGENT PHYSIOLOGICAL STATE RECOGNITION MODEL, METHOD AND APPARATUS FOR PHYSIOLOGICAL STATE RECOGNITION, AND DEVICE " and filed with China National Intellectual Property Administration on November 30, 2023, and Chinese Patent Application No. 202311810471.4, titled "METHOD FOR PROCESSING PHYSICAL AND PSYCHOLOGICAL STATE, AND MODEL AND APPARATUS FOR PROCESSING PHYSIOLOGICAL AND PSYCHOLOGICAL STATE" and filed with China National Intellectual Property Administration on December 26, 2023, the entire disclosures of which are incorporated herein by reference.

### FIELD

The present disclosure relates to the technical field of physiological signal recognition, and more particularly, to a method for training a human-factors intelligent physiological state recognition model, a method and apparatus for physiological state recognition, and a device.

### BACKGROUND

A physiological signal can reflect a mental state, an emotional state, a health state (such as presence or absence of a disease), and other physiological states of a subject. How to perform more accurate physiological state recognition based on a physiological signal of a user is a problem to be solved.

### SUMMARY

The present disclosure provides a method for training a human-factors intelligent physiological state recognition model, a method and apparatus for physiological state recognition, and a device, which can realize more accurate recognition of a physiological state of a subject, such as a mental state, an emotional state, or a health state.

In a first aspect, embodiments of the present disclosure provide a method for training a human-factors intelligent physiological state recognition model. The method includes: obtaining a first physiological signal, a first state label of the first physiological signal, a second physiological signal corresponding to the first physiological signal, and a second state label of the second physiological signal, in which the second physiological signal is a physiological signal generated based on the first physiological signal, the first physiological signal is a collected physiological signal, the first state label indicates a physiological state corresponding to the first physiological signal, and the second state label indicates a physiological state corresponding to the second physiological signal; and training the physiological state recognition model based on the first physiological signal, the second physiological signal, the first state label, and the second state label. In addition to using the collected real physiological signal, i.e., the first physiological signal, the method also uses the second physiological signal generated based on the first physiological signal to train the physiological state recognition model. Thus, an individualized difference in the physiological signals can be suppressed to some extent. Thus, a physiological state recognition model with a relatively higher accuracy can be obtained by training using relatively few real physiological signals. When using the physiological state recognition model to recognize the physiological state of the subject, such as the mental state, the emotional state, or the health state, accuracy of a recognition result is relatively higher.

In a second aspect, embodiments of the present disclosure provide a method for physiological state recognition. The method includes: obtaining a physiological signal of a user; inputting the physiological signal into a predetermined physiological state recognition model, in which the physiological state recognition model is configured to recognize a physiological state based on the physiological signal, and the predetermined physiological state recognition model is obtained by training with the method according to any one of the embodiments of the first aspect; and obtaining a physiological state recognition result outputted by the physiological state recognition model as a physiological state recognition result of the user. Since the physiological state recognition model with the relatively higher accuracy is obtained by training with the above method, the accuracy of the recognition result is relatively higher when using the physiological state recognition model to recognize the physiological state of the subject, such as the mental state, the emotional state, or the health state, in the method.

In a third aspect, embodiments of the present disclosure provide an apparatus for training a physiological state recognition model. The apparatus includes an obtaining module and a training module. The obtaining module is configured to obtain a first physiological signal, a first state label of the first physiological signal, a second physiological signal corresponding to the first physiological signal, and a second state label of the second physiological signal. The second physiological signal is a physiological signal generated based on the first physiological signal. The first physiological signal is a collected physiological signal. The first state label indicates a physiological state corresponding to the first physiological signal. The second state label indicates a physiological state corresponding to the second physiological signal. The training module is configured to train the physiological state recognition model based on the first physiological signal, the second physiological signal, the first state label, and the second state label.

In a fourth aspect, embodiments of the present disclosure provide an apparatus for physiological state recognition. The apparatus includes an obtaining module and a recognition module. The obtaining module is configured to obtain a physiological signal of a user. The recognition module is configured to: input the physiological signal into a predetermined physiological state recognition model, in which the physiological state recognition model is configured to recognize a physiological state based on the physiological signal, and the predetermined physiological state recognition model is obtained by training with the method according to any one of the embodiments of the first aspect; and obtain a physiological state recognition result outputted by the physiological state recognition model as a physiological state recognition result of the target object.

In a fifth aspect, embodiments of the present disclosure provide an electronic device. The electronic device includes a processor and a memory. The memory has one or more computer programs stored thereon. The one or more computer programs include instructions. The instructions, when executed by the processor, cause the electronic device to perform the method according to any one of the embodiments of the first aspect or the second aspect.

In a sixth aspect, embodiments of the present disclosure provide a computer-readable storage medium. The computer-readable storage medium has a computer program stored therein. The computer program, when executed by a computer, causes the computer to perform the method according to any one of the embodiments of the first aspect or the second aspect.

### BRIEF DESCRIPTION OF THE DRAWINGS

FIG. 1 is a schematic structural diagram of an electronic device according to an embodiment of the present disclosure.
FIG. 2 is a schematic flowchart of a method for training a human-factors intelligent physiological state recognition model according to an embodiment of the present disclosure.
FIG. 3 is a schematic structural diagram of a generative adversarial network, GAN, according to an embodiment of the present disclosure.
FIG. 4 is a schematic flowchart of a method for training a generator according to an embodiment of the present disclosure.
FIG. 5A is another schematic flowchart of a method for training a generator according to an embodiment of the present disclosure.
FIG. 5B is another schematic flowchart of a method for training a physiological state recognition model according to an embodiment of the present disclosure.
FIG. 5C is a schematic diagram of electrocardiogram signals generated by the trained generator according to an embodiment of the present disclosure.
FIG. 6 is a schematic flowchart of a method for physiological state recognition according to an embodiment of the present disclosure.
FIG. 7 is a schematic structural diagram of an apparatus for training a human-factors intelligent physiological state recognition model according to an embodiment of the present disclosure.
FIG. 8 is a schematic structural diagram of an apparatus for physiological state recognition according to an embodiment of the present disclosure.
FIG. 9 is a schematic diagram of data decomposition according to an embodiment of the present disclosure.
FIG. 10 is a schematic diagram of a physiological state recognition model according to an embodiment of the present disclosure.

### DETAILED DESCRIPTION OF THE EMBODIMENTS

Preferred embodiments of the present disclosure will be described below with reference to the accompanying drawings. It should be understood by those skilled in the art that these embodiments are intended to explain technical principles of the present disclosure only rather than limit the scope of the present disclosure.

In order to make the purposes, technical solutions, and advantages of the embodiments of the present disclosure clearer, the technical solutions in the embodiments of the present disclosure are described clearly and completely with reference to the accompanying drawings. Obviously, the embodiments described here are only some embodiments of the present disclosure and are not all embodiments of the present disclosure. Based on the embodiments of the present disclosure, other embodiments obtained by those skilled in the art without inventive labor are within scope of the present disclosure.

It should be noted that in the description of the present disclosure, terms such as "first" and "second" are for ease of description only and do not indicate or imply relative importance of the apparatus, element, or parameter, and therefore cannot be understood as a limitation on the present disclosure. In addition, "and/or" herein describes an association relationship between correlated objects, including three relationships. For example, "A and/or B" can mean A only, B only, or both A and B. In addition, the symbol "/" generally indicates an "or" relationship between the correlated objects preceding and succeeding the symbol, unless otherwise specified.

### Embodiment 1

Human-factors intelligence is developed by combining a human-factors engineering technology with an artificial intelligence (AI) technology. The human-factors engineering technology is engineering and designs of applying human psychological and physiological principles to products, processes, and systems. That is, it is a technology that designs and improves a human-machine-environment system in accordance with human characteristics. Machines can interact with individuals more intelligently by combining this technology with the artificial intelligence technology. For example, in some scenarios, a physiological state of the individual can be recognized based on a physiological signal of the individual.

The physiological signal, such as an electrocardiogram signal, can reflect the physiological state of the individual, such as a mental state, an emotional state, and a health status (such as presence or absence of a certain disease). How to perform more accurate physiological state recognition of a user based on a physiological signal of the user is a problem to be solved.

Analysis reveals that the physiological signal exhibits an individualized difference, and thus inhibiting the individualized difference of the physiological signal is a way to improve accuracy of physiological state recognition of a user.

To this end, embodiments of the present disclosure provide a method for training a human-factors intelligent physiological state recognition model. The trained physiological state recognition model can inhibit the individualized difference of the physiological signal to some extent, and thus a recognition result is relatively more accurate when using the physiological state recognition model to recognize a physiological state of a subject based on a physiological signal of the subject.

Further, embodiments of the present disclosure also provide a method for physiological state recognition, which can use the physiological state recognition model to recognize the physiological state of the subject based on the physiological signal of the subject. Since the physiological state recognition model can inhibit the individualized difference of the physiological signal to some extent, a recognition result of the method for the physiological state recognition according to the embodiments of the present disclosure can be relatively more accurate.

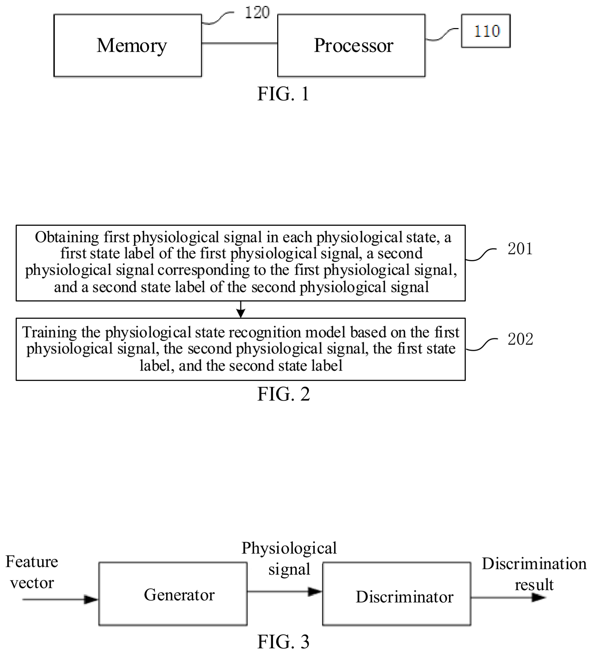

FIG. 1 is a schematic structural diagram of an electronic device according to an embodiment of the present disclosure. The electronic device 100 includes a processor 110, a memory 120, and the like.

In another exemplary embodiment, in order to improve functions of the electronic device, the electronic device may further include one or more devices of a display screen, a camera, a speaker, an antenna, a mobile communication module, a wireless communication module, an audio module, a telephone receiver, a microphone, a headphone interface, a charging management module, a power supply management module, or a battery, which is not limited in the embodiments of the present disclosure.

The processor 110 can include one or more processing units. For example, the processor 110 can include an application processor (AP), a modem processor, a graphics processing unit (GPU), an image signal processor (ISP), a controller, a video codec, a digital signal processor (DSP), a baseband processor, and/or a neural-network processing unit (NPU). Different processing units may be independent devices or may be integrated in one or more processors.

An electrical signal transmitted by the camera can be converted into a digital image signal by the ISP. The ISP can output the digital image signal to the DSP for processing. The DSP can convert the digital image signal into a standard image signal in a standard format, such as RGB and YUV.

The controller can generate an operation control signal based on an instruction operation code and a sequence signal to complete control of fetching and execution of instructions.

The processor 110 is further provided with a memory for storing instructions and data. In another exemplary embodiment, the memory in the processor 110 is a cache memory. The memory can store instructions or data that the processor 110 has just used or uses cyclically. If the processor 110 needs to use the instructions or the data again, the processor 110 can invoke the instructions or the data directly from the memory. In this way, repeated access is avoided, reducing a waiting time of the processor 110, thereby improving system efficiency.

The memory 120 can store a computer-executable program code including instructions. The memory 120 can include a program storage area and a data storage area. The program storage area can store an operating system, applications required for at least one function (such as a sound playback function and an image playback function), and the like. The data storage area can store data (such as audio data) created during use of the electronic device 100. In addition, the memory 120 can include high-speed random access memory, and can further include a nonvolatile memory, such as at least one disk storage device, a flash memory device, or a universal flash memory (UFS). The processor 110 is configured to execute various functional applications and data processing of the electronic device 100 by running instructions stored in the memory 120 and/or instructions stored in the memory disposed in the processor.

It should be noted that in the embodiment shown in FIG. 1, the memory 120 disposed in the electronic device 100 is taken as an example. In other embodiments of the present disclosure, the above memory 120 may not be disposed in the electronic device 100. In this case, the memory 120 can be connected to the electronic device 100 via an interface provided by the electronic device 100 and further be connected to the processor 110 in the electronic device 100.

The method for training the physiological state recognition model and the method for the physiological state recognition according to the embodiments of the present disclosure will be described in detail below with reference to the structure of the above electronic device. It can be understood that the electronic device in the embodiments of the present disclosure can be further configured to perform a human-factors intelligence-based vital sign detection method as shown in Embodiment 2 and/or a target object recognition-based human-factors information processing method as shown in Embodiment 3.

The method for training the human-factors intelligent physiological state recognition model according to the embodiments of the present disclosure can be performed by an electronic device with the above structure. The method for the physiological state recognition according to the embodiments of the present disclosure can also be performed by an electronic device with the above structure. It can be understood that the electronic device configured to perform the method for training the human-factors intelligent physiological state recognition model and the electronic device configured to perform the method for the physiological state recognition can be the same electronic device or different electronic devices.

The physiological signal in the embodiments of the present disclosure can include an electrocardiogram signal or an electroencephalogram signal.

The physiological state in the embodiments of the present disclosure can include a mental state, an emotional state, or a health state, etc. For example, the mental state can specifically include mental concentration, lack of mental concentration, etc., the emotional state can specifically include joy, calmness, depression, etc., and the healthy state can specifically include healthy, non-healthy, etc.

FIG. 2 is a schematic flowchart of a method for training a human-factors intelligent physiological state recognition model according to an embodiment of the present disclosure. As shown in FIG. 2, the method can include operations at steps 201 and 202.

At step 201, a first physiological signal in each physiological state, a first state label of the first physiological signal, a second physiological signal corresponding to the first physiological signal, and a second state label of the second physiological signal are obtained.

The first physiological signal is a physiological signal collected by a physiological detection device, such as an electrocardiometer and an electroencephalography device, i.e., a real physiological signal collected from the subject. The first state label indicates a physiological state corresponding to the first physiological signal.

In another exemplary embodiment, timing and objects for collection of the first physiological signal may be different based on different application fields of the physiological state recognition model. For example, if a physiological state recognition model is applied to the field of autonomous driving, such as in an autonomous driving assistance system, the first physiological signal can be obtained by using a portable bioinformatics collection device to collect a physiological signal of a driver during driving.

The second physiological signal is a physiological signal generated based on the first physiological signal. The second state label indicates a physiological state corresponding to the second physiological signal.

In another exemplary embodiment, the first state label of the first physiological signal is the same as the second state label of the second physiological signal corresponding to the first physiological signal. For example, if the physiological state is the mental state, when the first state label is the mental concentration, the second state label is the mental concentration.

In another exemplary embodiment, different values can be used to identify different physiological states. For example, assuming that the physiological states include three types, 1, 2, and 3 can be used to represent one physiological state, respectively.

It can be understood that the first physiological signal, the first state label, the second physiological signal, and the second state label described above have a correspondence. For ease of illustration, in the embodiments of the present disclosure, they are referred to as a set of signal data (the first physiological signal, the second physiological signal, the first state label, the second state label). For example, for a first physiological signal a1, it has a second physiological signal b1 and a first state label t1 corresponding thereto, and the second physiological signal b1 has a second state label p1 corresponding thereto. Correspondingly, they constitute the set of signal data (a1, b1, t1, p1). Correspondingly, a plurality of sets of signal data under each physiological state need to be obtained in this step. For example, assuming that the physiological state is a mental state, which is classified into mental concentration and lack of mental concentration, a plurality of sets of signal data in such a mental state of the mental concentration and a plurality of sets of signal data in such a mental state of the lack of mental concentration can be obtained in this step.

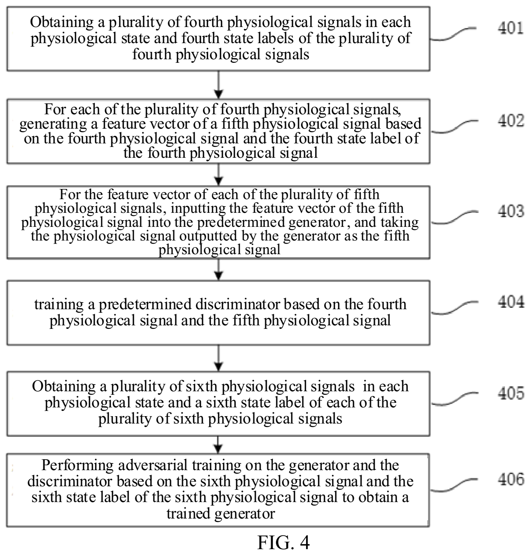

In an embodiment, a generator for generating the physiological signal can be pretrained. A method for training the generator can refer to specific description of an embodiment shown in FIG. 4, and details thereof are omitted herein. Then, the second physiological signal in this step can be a physiological signal generated by the generator. In an embodiment, the second physiological signal may be generated using the generator prior to the step 201 or may be generated in real time based on the first physiological signal when performing the step 201.

In another exemplary embodiment, when performing the step 201, if the second physiological signal is generated in real time based on the first physiological signal, the obtaining the second physiological signal corresponding to the first physiological signal at this step can include:
generating a feature vector of the second physiological signal based on the first physiological signal and the first state label of the first physiological signal; and
inputting the feature vector into a predetermined generator, and taking a physiological signal generated by the generator based on the feature vector as the second physiological signal corresponding to the first physiological signal.

In another exemplary embodiment, the generator can be composed of a combination of a convolutional (Conv) layer and a linear (Linear) layer, and the specific structure thereof is not limited in the present disclosure.

In another exemplary embodiment, the generating the feature vector of the second physiological signal based on the first physiological signal and the first state label of the first physiological signal can specifically include:
extracting a predetermined feature of the first physiological signal;
generating a feature vector of the first physiological signal based on the predetermined feature and the first state label of the first physiological signal; and
generating the feature vector of the second physiological signal based on the feature vector of the first physiological signal.

In another exemplary embodiment, the generating the feature vector of the second physiological signal based on the feature vector of the first physiological signal can specifically include: performing normalization processing on the feature vector of the first physiological signal to obtain the feature vector of the second physiological signal.

The first physiological signal being an electrocardiogram signal is taken as an example for illustration. In the electrocardiogram signal, a difference exists in a time interval between adjacent R waves, i.e., heart rate variability (HRV). That is, the HRV refers to a variation in differences between successive heartbeat cycles. Correspondingly, the predetermined feature at the above step can be an HRV feature of the electrocardiogram signal. The above extracting the predetermined feature of the first physiological signal can specifically be extracting the HRV feature of the first electrocardiogram signal.

In another exemplary embodiment, the HRV feature can specifically include an HRV time domain feature of the first electrocardiogram signal. In some embodiments, the HRV feature can further include an HRV frequency domain feature and/or an HRV nonlinear feature.

The HRV time domain feature can include a mean of RR intervals mean_nni, a standard deviation of RR intervals sdnn, a standard deviation of differences between adjacent RR intervals sdsd, the number of differences of continuous RR intervals greater than 50 ms nni_50, nni_50/the number of RR intervals pnni_50, the number of differences between continuous RR intervals greater than 20ms nni_20, nni_20/the number of RR intervals pnni_20, a square root of a mean of a sum of squared differences between adjacent RR intervals rmssd, an absolute value of a median of differences between RR intervals median _nni, a difference between a maximum RR interval and a minimum RR interval range_nni, a coefficient of variations of continuous differences cvcd, a coefficient of a variation cvnni, a mean heart rate mean_hr, a maximum heart rate max_hr, a minimum heart rate min_hr, and/or a standard deviation of heart rates std_hr.

The above HRV frequency domain feature can include a variance of HRV at a low frequency (0.04 hz to 0.15 hz) lf, a variance of HRV at a high frequency (0.15 hz to 40 hz) hf, a ratio of the variance of the HRV at the low frequency (0.04 hz to 0.15 hz) to the variance of the HRV at the high frequency (0.15 hz to 40 hz) lf/hf, normalized low frequency power lf_nu, normalized high frequency power hf_nu, a total power density spectrum total_power, a variance of HRV at an extremely low frequency (0.003 hz to 0.04 hz) vlf, and/or an HRV triangular index measurement value triangular_index.

The above HRV nonlinear feature can include a baseline width of a triangle-based distribution tinn, a short axis of an ellipse sd1, a long axis of an ellipse sd2, a ratio of the short axis of the ellipse and the long axis of the ellipse sd2/sd1, a cardiac sympathetic index csi, a cardiac vagal index cvi, and/or a modified cardiac sympathetic index, CSI, modified_csi, etc.

In another exemplary embodiment, the above extracting HRV feature of the first electrocardiogram signal can specifically include:
extracting RR interval information of the first electrocardiogram signal; and
extracting the HRV feature of the first electrocardiogram signal based on the RR interval information of the first electrocardiogram signal.

An RR interval refers to a distance (a time interval) between two adjacent R waves in the electrocardiogram signal. In an embodiment of the present disclosure, a time interval between peaks of two adjacent R waves is referred to as the RR interval. The R wave refers to a peak in the electrocardiogram signal.

In another exemplary embodiment, at this step, QRS waveforms in a first electrocardiogram signal can be detected, a position of an R wave peak in each QRS waveform can be extracted, a distance between each two adjacent R wave peaks can be calculated, and the distance between each two adjacent R wave peaks can be divided by a sampling rate to obtain an RR interval list of the first electrocardiogram signal. The RR interval list includes each RR interval RRI in the first electrocardiogram signal, where an i-th RR interval is denoted as *RRIᵢ,* which represents a time interval between an (i+1)-th R wave peak and an i-th R wave peak in the first electrocardiogram signal.

In another exemplary embodiment, the detecting the QRS waveforms in the first electrocardiogram signal can be implemented using a Pan-Tompkins algorithm.

The first physiological signal being an electroencephalogram signal is taken as an example for illustration. The above extracting the predetermined feature of the first physiological signal can be specifically extracting a time domain feature, a frequency domain feature, a time frequency feature, and/or a nonlinear feature of the electroencephalogram signal.

The time domain feature of the electroencephalogram signal can include an amplitude, a variance, a mean, a root mean square, a skewness, a peak of the electroencephalogram signal, etc.

The frequency domain feature of the electroencephalogram signal can include power, power spectral density, energy of the electroencephalogram signal in each of a delta band (0 Hz to 4 Hz), a theta band (4 to 8 Hz), an alpha band (8 Hz to 13 Hz), a beta band (13 Hz to 25 Hz), a gamma band (25 Hz to 50 Hz), etc.

The time-frequency feature of the electroencephalogram signal can include a frequency domain feature of the electroencephalogram signal of each time window among a plurality of time windows of the electroencephalogram signal obtained by a time-frequency analysis method. For the frequency domain feature of the electroencephalogram signal in each time window, reference can be made to the above examples of the frequency domain feature of the electroencephalogram signal, and details thereof are omitted herein. The above time-frequency analysis method can include short-time Fourier transform (STFT), wavelet transform (WT), Hilbert-Huang transform (HHT), etc.

The nonlinear feature of the electroencephalogram signal can include various types of entropy, a correlation dimension, a fractional dimension of the electroencephalogram signal, etc. The various types of the entropy of the electroencephalogram signal can include approximate entropy, wavelet entropy feature, asymmetric entropy feature, etc.

In another exemplary embodiment, the generating the feature vector of the first physiological signal based on the predetermined feature and the first state label of the first physiological signal can specifically include:
performing normalization processing on the first state label of the first physiological signal, and taking the normalized result as a label component of the first physiological signal; and
taking the predetermined feature and the label component of the first physiological signal as components in the feature vector, respectively, to generate the feature vector of the first physiological signal.

The first physiological signal being an electrocardiogram signal is still taken as an example for illustration. Assuming that the extracted HRV feature of the first electrocardiogram signal includes all of the above thirty-one HRV features, the above thirty-one features are denoted as *F₁, F₂, F₃, ..., F₃₁,* respectively, and the label component can be denoted as *F₃₂,* then a feature vector F of the first electrocardiogram signal can include a total of thirty-two components with the HRV features and the label component.

The above extracting the HRV feature of the first electrocardiogram signal based on the RR interval information of the first electrocardiogram signal can be implemented using related technologies, and details thereof are omitted in the embodiments of the present disclosure.

In another exemplary embodiment, if the physiological state includes n types and the first state label represents different physiological states by 1, 2, ..., n, then the normalization processing on the first state label can be the first state label/n. For example, if the physiological state is a mental state, which includes three types represented by 1, 2, and 3, respectively, then the normalized label component is 1/3 when the first state label is 1, the normalized label component is 2/3 when the first state label is 2, and the normalized label component is 1 when the first state label is 3.

At step 202, the physiological state recognition model is trained based on the first physiological signal, the second physiological signal, the first state label, and the second state label.

In another exemplary embodiment, for each set of the signal data (the first physiological signal, the second physiological signal, the first state label, the second state label), a training sample (the first physiological signal, the first state label) can be generated based on the first physiological signal and the first state label, and another training sample (the second physiological signal, the second state label) can be generated based on the second physiological signal and the second state label, and the physiological state recognition model is trained using the above two training samples.

In the above implementation, for each set of signal data (the first physiological signal, the second physiological signal, the first state label, the second state label), the two training samples including (the first physiological signal, the first state label) and (the second physiological signal, the second state label) can be generated. In another exemplary embodiment, for the two training samples corresponding to different sets of signal data, an order of inputting the above training samples into the physiological state recognition model can be shuffled in order to improve the accuracy of the physiological state recognition model obtained by the training, and thus an order of inputting the two training samples of one set of signal data into the physiological state recognition model is not consecutive. For example, assuming that there are two training samples generated based on one set of signal data including (the first physiological signal 1, the first state label) and (the second physiological signal 1, the second state label), and two training samples generated based on another set of signal data including (the first physiological signal 2, the first state label) and (the second physiological signal 2, the second state label), the order of inputting the above samples into the physiological state recognition model can be shuffled, for example, the order of inputting the above four samples into the physiological state recognition model is: a training sample (the first physiological signal 1, the first state label), a training sample (the second physiological signal 2, the second state label), a training sample (the second physiological signal 1, the second state label), and a training sample (the first physiological signal 2, the first state label). Thus, the order of inputting the training sample (the first physiological signal 1, the first state label) and the training sample (the second physiological signal 1, the second state label) into the physiological state recognition model is not consecutive, and the order of inputting the training sample (the first physiological signal 2, the first state label) and the training sample (the second physiological signal 2, the second state label) into the physiological state recognition model is not consecutive. It can be understood that the order of inputting the above four training samples into the physiological state recognition models is only an example. In actual applications, more training samples are generated based on more sets of signal data. As long as the order of inputting the two training samples corresponding to one set of signal data into the physiological state recognition models is not consecutive, the specific order of inputting a plurality of training samples into the physiological state recognition model is not limited in the embodiments of the present disclosure.

In another exemplary embodiment, the physiological state recognition model in the embodiments of the present disclosure can be implemented by a Bayesian neural network combined with a Transformer Encoder. In an embodiment, the physiological state recognition model according to the embodiments of the present disclosure can be that a parameter dimension calculated by each neuron in the Transformer Encoder is added based on the Transformer Encoder. For example, the calculated parameter is expanded from one dimension to two dimensions by fitting a mean and a variance of each neuron to obtain the above Bayesian neural network combined with the Transformer Encoder. The Bayesian neural network combined with the Transformer Encoder can be implemented based on a Bayesian neural network with a convolutional layer or a linear layer, which can realize a conversion from a point estimation of the parameter by the original Transformer Encoder into a parameter distribution estimation by the new Transformer Encoder, effectively reducing sensitivity of the physiological state recognition model to the individual difference. Moreover, for a physiological signal with a relatively long duration, long-term time-series dependency can be captured more effectively, effectively combining the characteristic of the physiological signal, such as the electrocardiogram signal, with the long-term time-series dependency, i.e., combining the "context" information of the physiological signal, to give a more accurate recognition result, thereby improving the recognition accuracy of the physiological state recognition model.

In another exemplary embodiment, in this step, one training sample or a plurality of training samples can be obtained once and inputted into a predetermined physiological state recognition model to train the physiological state recognition model. Forward propagation and backpropagation processes during the training are not limited in the embodiments of the present disclosure, which can be implemented with reference to the related art.

In the method for the physiological state recognition according to the embodiments of the present disclosure, in addition to using the collected real physiological signal to train the physiological state recognition model, the physiological signal generated from the real physiological signal is used to train the physiological state recognition model. Thus, relatively more physiological signals can be used to train the physiological state recognition model. In this way, a physiological state recognition model with relatively higher accuracy can be obtained by training with relatively few real physiological signals.

The generator for generating the physiological signal in the embodiments of the present disclosure can be trained in a generative adversarial manner. In an embodiment of the present disclosure, a GAN can be pre-established. As shown in FIG. 3, the GAN can include a generator and a discriminator, and a physiological signal generated by the generator can be inputted to the discriminator for discrimination. Each of the generator and discriminator described above can be implemented with a neural network. The generator and the discriminator that are initially established are untrained networks. The trained generator obtained by the method shown in FIG. 4 can be used as the generator for generating the second physiological signal in the embodiment of the present disclosure shown in FIG. 2.

As shown in FIG. 4, the generator is trained by operations at step 401 to 406.

At step 401, a plurality of fourth physiological signals in each physiological state and fourth state labels of the plurality of fourth physiological signals are obtained.

The fourth physiological signal is a physiological signal collected by a physiological detection device, i.e., a real physiological signal collected for the subject. The fourth state label indicates a physiological state of the fourth physiological signal.

The number of fourth physiological signals in each physiological state is not limited in the embodiments of the present disclosure. It can be understood that performance of the trained generator is better with an increased number of fourth physiological signals.

For the implementation of the fourth state label of the fourth physiological signal, reference can be made to the aforementioned description of the first state label and the second state label, and details thereof are omitted herein.

In another exemplary embodiment, the fourth physiological signal obtained at this step may be the same as or different from the first physiological signal at the step 201, as long as it is a real physiological signal.

At step 402, for each of the plurality of fourth physiological signals, a feature vector of a fifth physiological signal is generated based on the fourth physiological signal and the fourth state label of the fourth physiological signal.

For the implementation of this step, reference can made to the aforementioned description of the feature vector of the second physiological signal generated based on the first physiological signal and the first state label of the first physiological signal, with differences mainly in replacing the first physiological signal with the fourth physiological signal, the first state label with the fourth state label, and the second physiological signal with the fifth physiological signal, and details thereof are omitted herein.

At step 403, for the feature vector of each fifth physiological signal, the feature vector of the fifth physiological signal is inputted into the predetermined generator, and the physiological signal outputted by the generator is taken as the fifth physiological signal.

Through the processing at this step, the fifth physiological signal corresponding to each fourth physiological signals can be obtained.

In another exemplary embodiment, at this step, a feature vector of one fifth physiological signal can be obtained once and inputted into the predetermined generator to generate a fifth physiological signal correspondingly; or at this step, feature vectors of a plurality of fifth physiological signals can be obtained once and inputted into the predetermined generator to generate a plurality of fifth physiological signals correspondingly.

The generator of the embodiments of the present disclosure is configured to generate the physiological signal based on the feature vector of the physiological signal, and thus the physiological signal generated by the generator can be relatively more in line with expectations, improving availability of the generated data.

At step 404, a predetermined discriminator is trained based on the fourth physiological signal and the fifth physiological signal.

In another exemplary embodiment, this step can include:
assigning a first source label to the fourth physiological signal and a second source label to the fifth physiological signal, in which the first source label indicates that the fourth physiological signal is a real physiological signal, and the second source label indicates that the fifth physiological signal is a physiological signal generated by the generator; and
training the discriminator based on the fourth physiological signal, the first source label, the fifth physiological signal, and the second source label.

In another exemplary embodiment, the first source label and the second source label can be implemented by 1 and 0, respectively. That is, 1 represents that the physiological signal is the real physiological signal, and 0 represents that the physiological signal is the generated physiological signal.

The discriminator is configured to determine whether the received physiological signal is a real physiological signal or a generated physiological signal.

The above training the discriminator based on the fourth physiological signal, the first source label, the fifth physiological signal, and the second source label can specifically include:
generating a training sample of the discriminator based on the fourth physiological signal and the first source label of the fourth physiological signal, and generating another training sample of the discriminator based on the fifth physiological signal and the second source label of the fifth physiological signal; and
training the discriminator using the above training samples.

Based on the above processing steps, for each fourth physiological signal, two training samples can be generated correspondingly. It is assumed that the two training samples are denoted as (the fourth physiological signal, the first source label) and (the fifth physiological signal, the second source label). In some embodiments, in order to improve processing accuracy of the discriminator, when training the discriminator using the training samples, an order of inputting two training samples corresponding to one fourth physiological signal into the discriminator for training the discriminator is not consecutive. For example, assuming that a fourth physiological signal 1 corresponds to two training samples, which are (a fourth physiological signal 1, the first source label) and (a fifth physiological signal 1, the second source label), and the fourth physiological signal 2 corresponds to two training samples, which are (a fourth physiological signal 2, the first source label) and (a fifth physiological signal 2, the second source label), an order of training the discriminator using the above four training samples, for example, an order of inputting the above samples into the discriminator after shuffling is: a training sample (the fourth physiological signal 1, the first source label), a training sample (the fifth physiological signal 2, the second source label), a training sample (the fifth physiological signal 1, the second source label), and a training sample (the fourth physiological signal 2, the first source label). Thus, the order of inputting the training samples including (the fourth physiological signal 1, the first source label) and (the fifth physiological signal 1, the second source label) into the discriminator and the order of inputting the training samples including (the fourth physiological signal 2, the first source label) and (the fifth physiological signal 2, the second source label) into the discriminator can be not consecutive. It can be understood that the order of inputting the above four training samples into the discriminators is only an example. In actual applications, more training samples are generated with more fourth physiological signals. As long as the order of inputting the two training samples corresponding to one fourth physiological signal into the discriminator is not consecutive, the specific order of inputting a plurality of training samples into the discriminator is not limited in the embodiments of the present disclosure.

The specific implementation of training the discriminator using the training samples can be implemented using a relevant training method of the discriminator, and details thereof are omitted herein.

At step 405, a plurality of sixth physiological signals in each physiological state and a sixth state label of each of the plurality of sixth physiological signals are obtained.

The sixth physiological signal can be a physiological signal collected by a physiological detection device, i.e., a real physiological signal collected for the subject. The sixth state label indicates a physiological state of the sixth physiological signal.

The number of sixth physiological signals in each physiological state is not limited in the embodiments of the present disclosure. It can be understood that the performance of the trained generator is better with an increased number of sixth physiological signals.

For the implementation of the sixth state label of the sixth physiological signal, reference can be made to the aforementioned description of the first state label and the second state label, and details thereof are omitted herein.

In another exemplary embodiment, the sixth physiological signal obtained at this step may be the same as or different from the fourth physiological signal at the step 401, as long as it is a real physiological signal.

At step 406, adversarial training is performed on the generator and the discriminator based on the sixth physiological signal and the sixth state label of the sixth physiological signal to obtain a trained generator.

In another exemplary embodiment, this step can include:
generating, for each of the plurality of sixth physiological signals, a feature vector of a seventh physiological signal based on the sixth physiological signal and the sixth state label of the sixth physiological signal;
inputting the feature vector of each seventh physiological signal into the generator, and taking a physiological signal outputted by the generator correspondingly as the seventh physiological signal;
assigning a first source label to the sixth physiological signal and a second source label to the seventh physiological signal, in which the first source label indicates that the sixth physiological signal is a real physiological signal, and the second source label indicates that the seventh physiological signal is a physiological signal generated by the generator;
generating a training sample of the discriminator based on the sixth physiological signal and the first source label of the sixth physiological signal, and generating another training sample of the discriminator based on the seventh physiological signal and the second source label of the seventh physiological signal; and
inputting the training samples of the discriminator into the discriminator, and performing backpropagation of each of the generator and the discriminator based on a discrimination result of the discriminator, i.e., adjusting a parameter of each of the generator and the discriminator.

For the implementation of the first source label and the second source label described above, reference can be made to the above corresponding description, and details thereof are omitted herein.

In another exemplary embodiment, if the sixth physiological signal obtained at the step 405 is the same physiological signal as the fourth physiological signal at the step 401, the action of generating the feature vector of the seventh physiological signal in the above step can be omitted.

For the above implementation of generating the feature vector of the seventh physiological signal based on the sixth physiological signal and the sixth state label of the sixth physiological signal, reference can be made to the aforementioned relevant description of generating the feature vector of the second physiological signal based on the first physiological signal and the first state label of the first physiological signal, with differences mainly in replacing the first physiological signal with the sixth physiological signal, the first state label with the sixth state label, and the second physiological signal with the seventh physiological signal, and details thereof are omitted herein.

For the specific implementation of inputting the feature vector of the seventh physiological signal into the generator and taking the physiological signal generated by the generator correspondingly as the seventh physiological signal, reference can be made to the description at the step 403, with a difference mainly in replacing the fifth physiological signal with the seventh physiological signal.

The backpropagation of each of the generator and the discriminator based on the discrimination result output by the discriminator can be performed using related technologies, and details thereof are omitted in the embodiments of the present disclosure.

At this step, the generator and the discriminator can compete and cooperate with each other through adversarial training. The generator aims to generate a physiological signal which gets increasingly close to the real physiological signal, enabling the discriminator to fail to accurately determine a source of the physiological signal. The discriminator aims to accurately determine whether the input physiological signal is the physiological signal generated by the generator or the real physiological signal. By adjusting the parameter of each of the generator and discriminator in the adversarial training, the physiological signal generated by the trained generator can ultimately be made very close to the real physiological signal, and the discriminator cannot accurately distinguish between the generated physiological signal and the real physiological signal.

In the embodiments of the present disclosure, the generator is trained through the generative adversarial training, and thus the generator can be used to generate the physiological signal very close to the real physiological signal for training the physiological state recognition model, which increases sample data volume for training the physiological state recognition model and increases diversity of the sample data volume. Therefore, the sensitivity of the physiological state recognition model to the individual differences in physiological signals can be effectively reduced when training the physiological state recognition model.

The method for training the generator is illustrated below by taking as an example a case where the physiological signal is an electrocardiogram signal, the physiological state is a mental state, and the mental state is classified into three types.

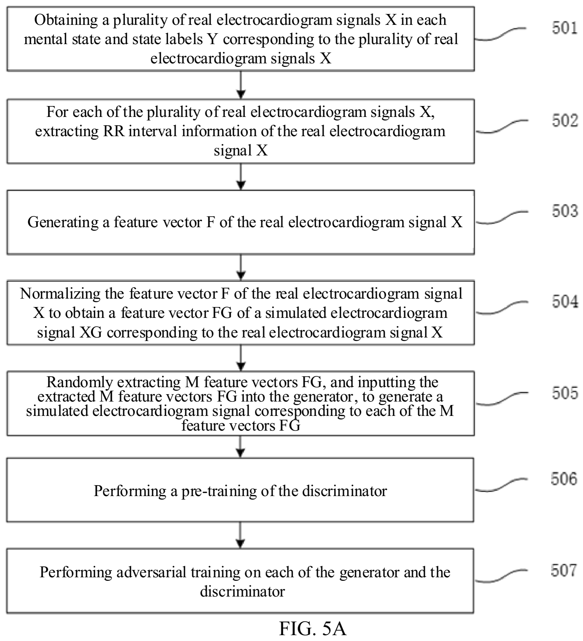

As shown in FIG. 5A, the method can include operations at steps 501 to 507.

At step 501, a plurality of real electrocardiogram signals X and state labels Y corresponding to the plurality of real electrocardiogram signals X in each mental state are obtained.

The following steps 502 to 504 are performed for each of the plurality of real electrocardiogram signals X.

At step 502, for each of the plurality of real electrocardiogram signals X, RR interval information of the real electrocardiogram signal X is extracted.

At step 503, a feature vector F of the real electrocardiogram signal X is generated.

At this step, the HRV feature of the real electrocardiogram signal X can be extracted based on the RR interval information of the real electrocardiogram signal X as a component in the feature vector F of the real electrocardiogram signal X, and the mental state label Y of the real electrocardiogram signal X can be normalized as a label component *F₃₂* in the feature vector F of the real electrocardiogram signal X.

Assuming that the HRV feature component includes all of the thirty-one HRV features in the above examples, the feature vector F of the real electrocardiogram signal X may include the above thirty-one HRV feature components, which are denoted as *F₁, F₂, F₃, ..., F₃₁,* respectively.

For example, assuming that the electrocardiogram signal-based mental state includes three types, the mental state label Y of the mental state of a first type can be denoted as 1, the mental state label Y of the mental state of a second type can be denoted as 2, and the mental state label Y of the mental state of a third type can be denoted as 3; the normalized mental state label *Y_{scaled-1}* of the mental state of the first type can be denoted as 1/3, the normalized mental state label *Y_{scaled-2}* of the mental state of the second type can be denoted as 2/3, and the normalized mental state label *Y_{scaled-3}* of the mental state of the third type can be denoted as 1.

Through the processing at this step, for each real electrocardiogram signal X, the feature vector F thereof can include a total of thirty-two components *F₁* to *F₃₂.*

At step 504, the feature vector F of the real electrocardiogram signal X is normalized to obtain a feature vector FG of a simulated electrocardiogram signal XG corresponding to the real electrocardiogram signal X.

The simulated electrocardiogram signal refers to an electrocardiogram signal generated based on the real electrocardiogram signal.

For example, taking the feature vector F of the real electrocardiogram signal X including the thirty-two components as an example, the feature vector FG of a to-be-generated electrocardiogram signal XG also includes thirty-two components, which are assumed to be denoted as *FG₁, FG₂, FG₃, ..., FG₃₂.* The feature component *FGᵢ* is a processing result obtained by normalizing the component *Fᵢ* in the first feature vector F, where i ranges from 1 to 32.

By performing the above steps 502 to 505 for each real electrocardiogram signal X, the feature vector FG of the to-be-generated electrocardiogram signal XG corresponding to the real electrocardiogram signal X can be obtained. That is, assuming that N real electrocardiogram signals X are provided, N feature vectors FG can be obtained.

At step 505, M feature vectors FG are randomly extracted, and the extracted M feature vectors FG are inputted into the generator, to generate a simulated electrocardiogram signal XG corresponding to each of the M feature vectors FG.

M is a natural number, and M<N. The specific value of M is not limited in the embodiments of the present disclosure.

It should be noted that each feature vector FG can be extracted only once, i.e., inputted into the generator only once, as long as the simulated electrocardiogram signal XG corresponding to each feature vector FG is generated.

Through the processing at this step, N simulated electrocardiogram signals XG corresponding to the N feature vectors FG can be obtained.

At step 506, a pre-training of the discriminator is performed.

At this step, a first source label L1 can be assigned to each real electrocardiogram signal X to obtain a real electrocardiogram sample YR (X, L1), and a second source label L2 can be assigned to each simulated electrocardiogram signal XG to obtain a simulated electrocardiogram sample YG (XG, L2). The real electrocardiogram sample YR and the simulated electrocardiogram sample YG can be randomly inputted into the discriminator separately for training the discriminator to obtain the trained discriminator. In another exemplary embodiment, when the real electrocardiogram sample YR and the simulated electrocardiogram sample YG are inputted into the discriminator, an order of inputting the real electrocardiogram sample YR corresponding to one real electrocardiogram signal X and the simulated electrocardiogram sample YG corresponding to the same real electrocardiogram signal X into the discriminator is not consecutive.

The first source label L1 indicates that the electrocardiogram signal is real data, and the second source label L2 indicates that the electrocardiogram signal is generated data. For example, the first source label L1 can be denoted as 1, and the second source label L2 can be denoted as 0. Then, the above real electrocardiogram sample YR may be denoted as (X, 1), and the above simulated electrocardiogram sample YG may be denoted as (XG, 0).

At step 507, adversarial training is performed on each of the generator and the discriminator.

In a first example, if the plurality of real electrocardiogram signals X obtained at the step501 are still used for the adversarial training at this step, the feature vector FG can be inputted into the generator to obtain the simulated electrocardiogram signal XG generated by the generator. The first source label L1 is assigned to the real electrocardiogram signal X, to obtain an adversarial training sample XF (X, L1). The second source label L2 is assigned to the simulated electrocardiogram signal XG, to obtain an adversarial training sample YF (XG, L2). The adversarial training samples XF (X, L1) and YF (XG, L1) are inputted into the discriminator to obtain corresponding discrimination results outputted by the discriminator, respectively. Backpropagation of each of the generator and the discriminator is performed based on the discrimination results. The above training process is repeated until the electrocardiogram signal XG generated by the generator is close enough to the real electrocardiogram signal and therefore a predetermined training stop condition is satisfied.

In a second example, if real electrocardiogram signals other than the real electrocardiogram signals at the step 501 can be used for the adversarial training at this step, the steps 502 to 504 can be performed for each real electrocardiogram signal to obtain a feature vector of each real electrocardiogram signal. Then, in accordance with a similar method as in the first example, a step, for example, of inputting the feature vector into the generator is performed to implement the adversarial training of each of the generator and the discriminator.

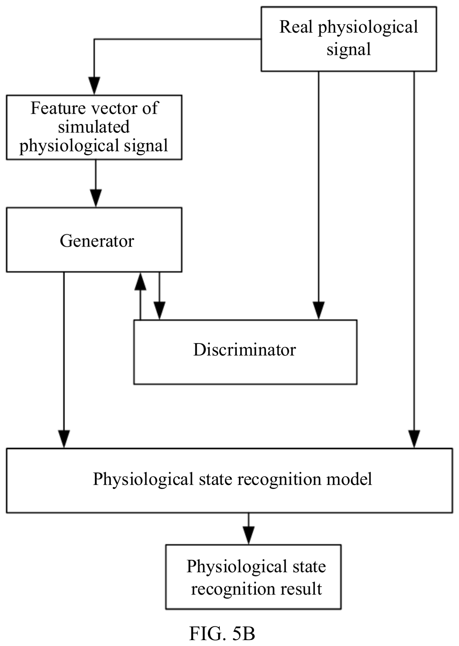

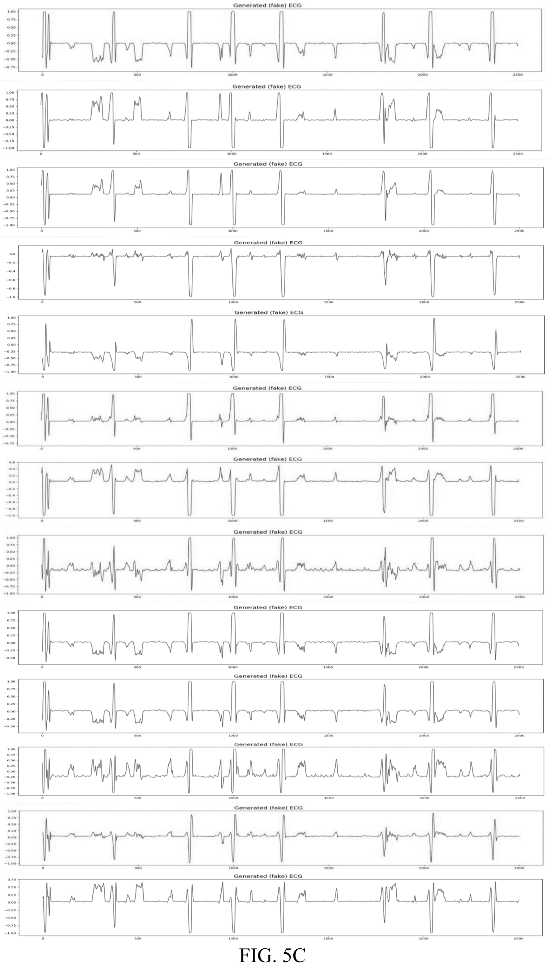

In combination with the above embodiments, referring to a schematic flowchart shown in FIG. 5B, in an embodiment provided by the present disclosure, a feature vector of the simulated physiological signal can be generated based on the real physiological signal and inputted into the generator, and the generative adversarial training can be performed on each of the generator and the discriminator based on the simulated physiological signal generated by the generator and the real physiological signal to obtain the trained generator. After that, a feature vector of the simulated physiological signal is generated based on the real physiological signal and inputted into the trained generator. A near-real simulated physiological signal is outputted by the generator, and the physiological state recognition model is trained based on the real physiological signal and the simulated physiological signal corresponding to the real physiological signal. Referring to FIG. 5C, FIG. 5C is a schematic diagram of 12-lead electrocardiogram signals generated by a trained generator according to an embodiment of the present disclosure.

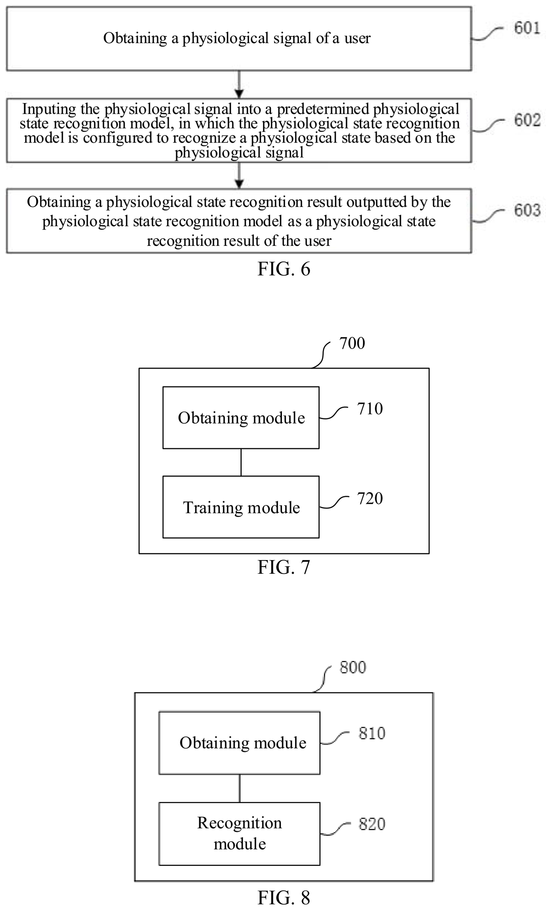

FIG. 6 is a schematic flowchart of a method for physiological state recognition according to an embodiment of the present disclosure. The method can be performed by an electronic device, for example, an on-board device for recognizing a physiological state of a driver, a medical device for recognizing a health state of a patient, etc. As shown in FIG. 6, the method can include operations at steps 601 to 603.

At step 601, a physiological signal of a user is obtained.

The above user can be, for example, a driver or medical personnel, and different implementations can occur based on different applicable scenarios of the method for the physiological state recognition.

At step 602, the physiological signal is inputted into a predetermined physiological state recognition model. The physiological state recognition model is configured to recognize a physiological state based on the physiological signal.

In another exemplary embodiment, the physiological state recognition model used at this step is a physiological state recognition model obtained by training with the aforementioned method.

At step 603, a physiological state recognition result outputted by the physiological state recognition model is obtained as a physiological state recognition result of the user.

Since the physiological state recognition model according to the embodiments of the present disclosure can inhibit the individualized differences in the physiological signals and has the relatively more accurate physiological state recognition result, the method for the physiological state recognition according to the embodiments of the present disclosure can also more accurately realize recognition of the physiological state of the user.

FIG. 7 is a schematic structural diagram of an apparatus for training a human-factors intelligent physiological state recognition model according to an embodiment of the present disclosure. As shown in FIG. 7, the apparatus 700 can include an obtaining module 710 and a training module 702. The obtaining module 710 is configured to obtain a first physiological signal, a first state label of the first physiological signal, a second physiological signal corresponding to the first physiological signal, and a second state label of the second physiological signal. The second physiological signal is a physiological signal generated based on the first physiological signal. The first physiological signal is a collected physiological signal. The first state label indicates a physiological state corresponding to the first physiological signal. The second state label indicates a physiological state corresponding to the second physiological signal. The training module 720 is configured to train the physiological state recognition model based on the first physiological signal, the second physiological signal, the first state label, and the second state label.

FIG. 8 is a schematic structural diagram of an apparatus for physiological state recognition according to an embodiment of the present disclosure. As shown in FIG. 8, the apparatus 800 can include an obtaining module 810 and a recognition module 820. The obtaining module 810 is configured to obtain a physiological signal of a user. The recognition module 820 is configured to: input the physiological signal into a predetermined physiological state recognition model, in which the physiological state recognition model is configured to recognize a physiological state based on the physiological signal, and the predetermined physiological state recognition model is obtained by training with the method according to the above embodiments; and obtain a physiological state recognition result outputted by the physiological state recognition model as a physiological state of the user.

The apparatuses according to the embodiments shown in FIGS. 7 and 8 can be used to perform the technical solutions of the method embodiments of the present disclosure, and for their implementation principles and technical effects, reference can be made to the relevant description in the method embodiments.

It should be understood that the modules of the apparatuses shown in FIGS. 7 and 8 above are merely divided according to logic functions, and can be entirely or partially integrated into a physical entity or physically separated from each other in an actual implementation. These modules can all be implemented in a form of software invoked by a processing element; or can all be implemented in a form of hardware; or can be partially implemented in the form of software invoked by the processing element and partially implemented in the form of hardware. For example, the recognition module may be a processing element set up alone, or may be integrated in one of the chips of the electronic device. Other modules are implemented in a similar way to that of the recognition module. Furthermore, all or some of these modules may be integrated together or may be implemented independently. In an implementation, operations of the method described above or the above modules may be accomplished by an integrated logic circuit in hardware in the processing element or by instructions in the form of software.

### Embodiment 2

Physiological data refers to data generated when a human body acts and can represent a physiological state of an individual to some extent. The physiological data include, for example, electroencephalogram data, electrodermal activity data, and electrocardiogram data. The physiological state includes, for example, a fatigue state, an emotional state, and a load-bearing state of a subject.

Exemplarily, embodiments of the present disclosure provide a method for physiological state recognition. The method includes, for example, operations at steps 1 to 5.

At step 1, a plurality of pieces of physiological time-series data are obtained.

Exemplarily, the physiological data includes, for example, electroencephalogram data, electrodermal activity data, and electrocardiogram data. The electroencephalogram data is taken as an example for illustration, which can be collected by an electroencephalogram electrode. Since the electroencephalogram data is typically a kind of fluctuation data over time, it can be regarded as time-series data (referred as to sequential data). The physiological time-series data is obtained by collecting the physiological data. Each piece of physiological time-series data is collected through a corresponding data collection channel. For ease of understanding, sixteen data collection channels in the present disclosure are taken as an example for illustration. Each of the sixteen data collection channels corresponds to an electrode. Sixteen electrodes are attached to different positions on a human head for data collection. Electroencephalogram data is collected through the sixteen collection channels to obtain sixteen pieces of physiological time-series data (the electroencephalogram data).

At step 2, feature decomposition is performed on each of the plurality of pieces of physiological time-series data to obtain subsequence data for each of the plurality of pieces of physiological time-series data.

Exemplarily, each of the plurality of pieces of physiological time-series data implies a feature that conforms to a certain variation pattern. Therefore, it is necessary to perform the feature decomposition on each of the plurality of pieces of physiological time-series data. Each of the plurality of pieces of physiological time-series data can be decomposed to obtain a plurality of pieces of subsequence data, where each of the plurality of pieces of subsequence data represents, for example, a variation pattern.

At step 3, feature extraction is performed on the subsequence data corresponding to each of the plurality of pieces of physiological time-series data to obtain initial feature data.

For a plurality of pieces of subsequence data corresponding to each of the plurality of pieces of physiological time-series data, the feature extraction is performed on the plurality of pieces of subsequence data to obtain the initial feature data. The plurality of pieces of physiological time-series data in one-to-one correspondence with a plurality of pieces of initial feature data.

At step 4, a plurality of pieces of initial feature data in one-to-one correspondence with the plurality of pieces of physiological time-series data are fused to obtain fused feature data.

The physiological time-series data collected by data collection channels is different. For example, the physiological time-series data collected by some channels contains richer information or can better reflect the physiological state of the user. The fused feature data contains richer information by infusing initial feature data corresponding to a plurality of channels.

At step 5, the physiological state is predicted based on the fused feature data.

Exemplarily, the physiological state includes, for example, a fatigue state, an emotional state, a load-bearing state, an anxiety state, and a relaxation state of the subject. In the embodiments of the present disclosure, the subsequence data representing the variation pattern of the physiological time-series data is obtained by decomposing the physiological time-series data. Then, the feature extraction is further performed on the subsequence data to obtain the richer initial feature data. Considering the different data collected by the different channels, the initial feature data of the different channels are fused, and the physiological state is then predicted. In this way, prediction accuracy of the physiological state is improved.

It can be understood that the physiological time-series data obtained in Embodiment 2 of the present disclosure belongs to the physiological signal in Embodiment 1 above. That is, the physiological signal in Embodiment 1 can include a plurality of pieces of physiological time-series data. Thus, the physiological state recognition processing such as the prediction can be performed on the physiological signal according to the technical solutions of the present disclosure.

In another example, feature decomposition can be performed on each of the plurality of pieces of physiological time-series data to obtain subsequence data for each of the plurality of pieces of physiological time-series data.

For example, the feature decomposition can be performed on each of the plurality of pieces of physiological time-series data to obtain trend subsequence data and periodic subsequence data. The rend subsequence data represents a changing trend of the physiological time-series data over time, and the periodic subsequence data represents a periodic variation characteristic of the physiological time-series data. The periodic subsequence data can also be referred to as seasonal subsequence data. For example, the physiological time-series data is illustrated by taking the electrocardiogram data as an example. The electrocardiogram data represents a heartbeat of the user to some extent, and the periodic subsequence data can reflect a heartbeat cycle.

At least one of the trend subsequence data or the periodic subsequence data is then taken as the subsequence data for each of the plurality of pieces of physiological time-series data. For example, the trend subsequence data and the periodic subsequence data can be taken as the subsequence data.

Addition decomposition or multiplication decomposition can be used when performing the feature decomposition. For the obtained trend subsequence data and periodic subsequence data using the addition decomposition, the physiological time-series data is obtained by adding the trend subsequence data and the periodic subsequence data. For the obtained trend subsequence data and periodic subsequence data using the multiplication decomposition, the physiological time-series data is obtained by multiplying the trend subsequence data by the periodic subsequence data.

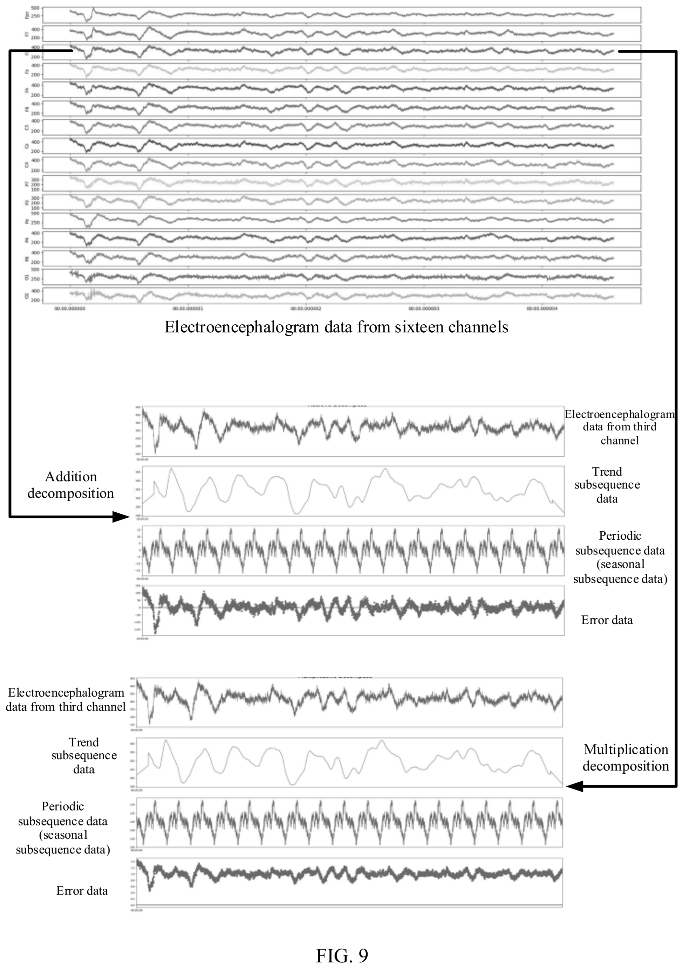

FIG. 9 is a schematic diagram of data decomposition according to an embodiment of the present disclosure.

As shown in FIG. 9, the physiological time-series data being electroencephalogram data from sixteen channels is taken as an example for illustration, in which feature decomposition is performed on each of sixteen pieces of physiological time-series data. FIG. 9 shows that trend subsequence data, periodic subsequence data (seasonal subsequence data), and error data obtained by performing the feature decomposition on electroencephalogram data from a third channel, where the error data is, for example, white noise. The error data can be removed, and the trend subsequence data and the periodic subsequence data can be retained as subsequence data for each piece of physiological time-series data.

Addition decomposition or multiplication decomposition can be used when performing feature decomposition. For the obtained trend subsequence data, periodic subsequence data, and error data using the addition decomposition, the physiological time-series data can be obtained by adding the trend subsequence data, the periodic subsequence data, and the error data can obtain. For the obtained trend subsequence data, periodic subsequence data, and error data using the multiplication decomposition, the physiological time-series data can be obtained by multiplying the trend subsequence data, the periodic subsequence data, and the error data.

It can be understood that the addition decomposition method or the multiplication decomposition can be selected according to actual conditions in the present disclosure. The subsequence data representing the variation pattern of the physiological time-series data can be obtained by decomposing the physiological time-series data, and then the feature extraction is further performed on the subsequence data to obtain the richer initial feature data. Compared with directly extracting the physiological time-series data, the feature extraction performed on the subsequence data after decomposing in the present disclosure can better extract an in-depth important feature. This indicates that an effect of the feature extraction is improved by performing the feature extraction subsequent to the data decomposition. Thus, accuracy of the physiological state processing is improved.

In another example, the feature of the subsequence data can be extracted using a neural network. For example, the subsequence data corresponding to each of the plurality of pieces of physiological time-series data is inputted into a neural network corresponding to each of the plurality of pieces of physiological time-series data for the feature extraction to obtain the initial feature data.

The neural network includes, for example, a long-term memory network (LSTM) and a bidirectional long short-term memory network (BiLSTM), and can also be other networks that can perform the feature extraction on the time-series data.

As a deep learning method, the neural network has powerful signal processing and recognition capabilities and can be applied in the field of physiological signals analysis. When using the neural network to detect the physiological signal, it is first necessary to select an appropriate network structure and configure corresponding parameters to learn and train the physiological data. Compared with a traditional machine learning algorithm that requires manual feature extraction and screening, the neural network can automatically perform feature selection and extraction on the inputted physiological data, reducing labor costs and improving the effect of the feature extraction.

In another example, when fusing the plurality of pieces of initial feature data, they can be fused based on a weight of the neural network. For example, for a plurality of neural networks in one-to-one correspondence with the plurality of pieces of physiological time-series data, a plurality of weights in one-to-one correspondence with the plurality of neural networks are determined. The plurality of weights forms a weight vector. Then, the plurality of pieces of initial feature data in one-to-one correspondence with the plurality of pieces of physiological time-series data are concatenated to obtain a concatenated vector. Finally, the fused feature data is obtained by multiplying the concatenated vector by the weight vector.

It can be understood that physiological time-series data from sixteen channels is taken as an example for illustration, in which the physiological time-series data from the sixteen channels corresponds to sixteen neural networks, and each of the sixteen neural networks corresponds to a weight. Different neural networks may correspond to different weights. For example, physiological time-series data collected by some of the sixteen channels contains richer information, and a weight corresponding to the physiological time-series data collected by the channels is larger when predicting the physiological state, thereby improving the prediction accuracy. For example, when collecting the electroencephalogram data, a local area of a brain is relatively active, and a channel corresponding to the area can collect richer and more abundant electroencephalogram data through the electrode. Therefore, a weight of the neural network corresponding to the channel should be larger.

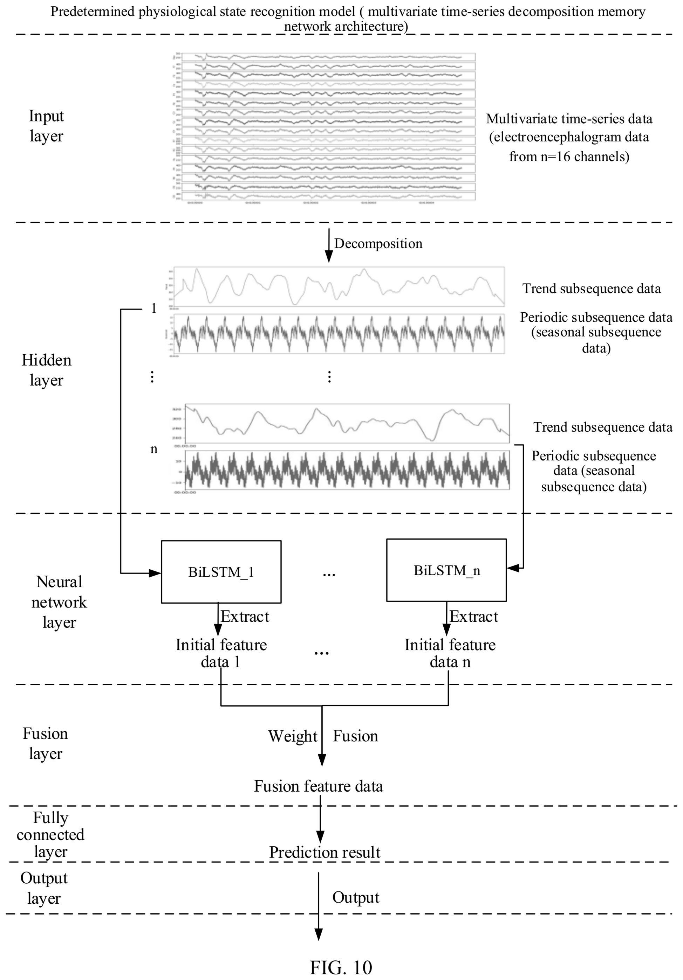

FIG. 10 is a schematic diagram of a physiological state recognition model according to an embodiment of the present disclosure.

As shown in FIG. 10, the physiological state recognition model includes, for example, an input layer, a hidden layer, a neural network layer, a fusion layer, a fully connected layer, and an output layer. The physiological state recognition model includes a multivariate time-series decomposition memory network architecture.

The input layer is configured to input the plurality of pieces of physiological time-series data that is collected. Each of the plurality of pieces of physiological time-series data is collected through a corresponding data collection channel. The physiological time-series data includes, for example, electroencephalogram data, which is a kind of multivariate time-series data.

The hidden layer is configured to perform feature decomposition on each of a plurality of pieces of physiological time-series data to obtain subsequence data for each of the plurality of pieces of physiological time-series data.

The neural network layer is configured to perform feature extraction on the subsequence data corresponding to each of the plurality of pieces of physiological time-series data to obtain initial feature data. For example, when the physiological time-series data is collected through n=16 channels, the physiological time-series data includes n=16 pieces, and each of the sixteen pieces of physiological time-series data corresponds to a set of subsequence data (including, for example, trend subsequence data and periodic subsequence data), thereby obtaining n=16 sets of subsequence data. The neural network layer includes sixteen neural networks in one-to-one correspondence with the sixteen sets of subsequence data, for example, BiLSTM_1 to BiLSTM_n. Each of the sixteen neural networks is configured to perform feature extraction on the corresponding subsequence data to obtain initial feature data 1 to initial data feature n in one-to-one correspondence with the sixteen neural networks.

The fusion layer is configured to fuse a plurality of pieces of initial feature data in one-to-one correspondence with the plurality of pieces of physiological time-series data to obtain fused feature data. When fusing the plurality of pieces of initial feature data, they can be fused based on a weight of the neural network. For example, for a plurality of neural networks in one-to-one correspondence with the plurality of pieces of physiological time-series data, a plurality of weights in one-to-one correspondence with the plurality of neural networks is determined. The plurality of weights forms a weight vector. Then, the plurality of pieces of initial feature data in one-to-one correspondence with the plurality of pieces of physiological time-series data are concatenated to obtain a concatenated vector. Finally, the fused feature data is obtained by multiplying the concatenated vector by the weight vector.

The fully connected layer is configured to predict the physiological state based on the fused feature data to obtain a prediction result. Alternatively, the physiological state recognition model can be configured to classify the physiological state, and the fully connected layer is configured to obtain a classification result based on the fused feature data. The classification categories include, for example, a fatigue state (fatigued, non-fatigued, fatigue level), an emotional state (happy, sad), and a load-bearing state (high load, low load, load level) of the subject.

The output layer is configured to output a prediction result or a classification result of the physiological state.

Exemplarily, embodiments of the present disclosure provide a method for training a physiological state recognition model. The method includes, for example, operations at steps 1 to 5. The physiological state recognition model includes a hidden layer, a neural network layer, a fusion layer, and a fully connected layer.

At step 1, feature decomposition is performed on each of a plurality of pieces of physiological time-series data by using the hidden layer to obtain subsequence data for each of the plurality of pieces of physiological time-series data.

Exemplarily, each of the plurality of pieces of physiological time-series data is collected through a corresponding data collection channel. The plurality of pieces of physiological time-series data is, for example, sample data for training the model. Sixteen data collection channels are taken as an example for illustration, where sixteen pieces of physiological time-series data collected by the sixteen data collection channels serve as a piece of sample data. The piece of sample data, for example, corresponds to sample label data. The sample label data, for example, represents a physiological state of the sample data.

At step 2, feature extraction is performed on the subsequence data corresponding to each of the plurality of pieces of physiological time-series data by using a neural network corresponding to each of the plurality of pieces of physiological time-series data to obtain initial feature data.

At step 3, a plurality of pieces of initial feature data in one-to-one correspondence with the plurality of pieces of physiological time-series data are fused by using the fusion layer to obtain fused feature data.

At step 4, the physiological state processing is performed based on the fused feature data by using the fully connected layer to obtain the prediction result.

At step 5, model parameters of the physiological state recognition model are adjusted based on an error between the prediction result and the sample label data, to train the physiological state recognition model.

For example, the model parameters of the physiological state recognition model are adjusted through backpropagation based on the error between the prediction result and the sample label data. The model parameters of the physiological state recognition model include, for example, a parameter of each of the hidden layer, the neural network layer, the fusion layer, and the fully connected layer.

In an example, the model parameters of the physiological state recognition model include a weight of the neural network corresponding to each of the plurality of pieces of physiological time-series sample data.

Exemplarily, the performing feature extraction on the subsequence data corresponding to each of the plurality of pieces of physiological time-series data by using the neural network corresponding to each of the plurality of pieces of physiological time-series data to obtain the initial feature data includes: determining, for a plurality of neural networks in one-to-one correspondence with the plurality of pieces of physiological time-series data, a plurality of weights in one-to-one correspondence with the plurality of neural networks, in which the plurality of weights forms a weight vector; concatenating the plurality of pieces of initial feature data in one-to-one correspondence with the plurality of pieces of physiological time-series data to obtain a concatenated vector; and obtaining the fused feature data by multiplying the concatenated vector by the weight vector.

The weight can be initially a default configuration prior to the model training. During a plurality of training epochs, the adjusting the model parameters of the physiological state recognition model through the backpropagation based on the error between the prediction result and the sample label data includes: adjusting the weights. After the model training is completed, the weight is determined. When using the trained physiological state recognition model to predict the physiological state, the physiological state is predicted based on the determined weight.

Exemplarily, embodiments of the present disclosure provide an apparatus for physiological state recognition. The apparatus includes, for example, an obtaining module, a first decomposition module, a first extraction module, a first fusion module, and a first prediction module.

Exemplarily, the obtaining module is configured to obtain the plurality of pieces of physiological time-series data. Each of the plurality of pieces of physiological time-series data is collected through a corresponding data collection channel.

Exemplarily, the first decomposition module is configured to perform feature decomposition on each of a plurality of pieces of physiological time-series data to obtain subsequence data for each of the plurality of pieces of physiological time-series data.

Exemplarily, the first extraction module is configured to perform feature extraction on the subsequence data corresponding to each physiological time-series data to obtain initial feature data.

Exemplarily, the first fusion layer is configured to fuse a plurality of pieces of initial feature data in one-to-one correspondence with the plurality of pieces of physiological time-series data to obtain fused feature data.

Exemplarily, the first prediction module is configured to predict the physiological state based on the fusion feature data.

Exemplarily, the first decomposition module is further configured to: perform the feature decomposition on each of the plurality of pieces of physiological time-series data to obtain trend subsequence data and periodic subsequence data, in which the trend subsequence data represents a changing trend of the physiological time-series data over time, and the periodic subsequence data represents a periodic variation characteristic of the physiological-time series data; and take at least one of the trend subsequence data or the periodic subsequence data as the subsequence data for each of the plurality of pieces of physiological time-series data.

Exemplarily, the physiological time-series data is obtained by adding the trend subsequence data and the periodic subsequence data or by multiplying the trend subsequence data by the periodic subsequence data.

Exemplarily, the first decomposition module is further configured to: perform the feature decomposition on each of the plurality of pieces of physiological time-series data to obtain trend subsequence data, periodic subsequence data, and error data; and remove the error data, and take trend subsequence data and periodic subsequence data as the subsequence data for each of the plurality of pieces of physiological time-series data.

Exemplarily, the first extraction module is further configured to input the subsequence data corresponding to each of the plurality of pieces of physiological time-series data into a neural network corresponding to each of the plurality of pieces of physiological time-series data for the feature extraction to obtain the initial feature data.

Exemplarily, the first fusion module is further configured to: determine, for a plurality of neural networks in one-to-one correspondence with the plurality of pieces of physiological time-series data, a plurality of weights in one-to-one correspondence with the plurality of neural networks, in which the plurality of weights forms a weight vector; concatenate the plurality of pieces of initial feature data in one-to-one correspondence with the plurality of pieces of physiological time-series data to obtain a concatenated vector; and obtain the fused feature data by multiplying the concatenated vector by the weight vector.

It can be understood that for the specific implementation process of apparatus for the physiological state recognition, reference can be made to the implementation process of the method for the physiological state recognition mentioned above, and details thereof are omitted herein.

Exemplarily, embodiments of the present disclosure provide an apparatus for training a physiological state recognition model. The apparatus includes a second decomposition module, a second extraction module, a second fusion module, a second prediction module, and an adjustment module.

Exemplarily, the second decomposition module is configured to perform, by using the hidden layer, feature decomposition on each of a plurality of pieces of physiological time-series data to obtain subsequence data for each of the plurality of pieces of physiological time-series data. Each of the plurality of pieces of physiological time-series data is collected through a corresponding data collection channel.

Exemplarily, the second extraction module is configured to perform, by using a neural network corresponding to each of the plurality of pieces of physiological time-series data, feature extraction on the subsequence data corresponding to each of the plurality of pieces of physiological time-series data to obtain initial feature data.

Exemplarily, the second fusion module is configured to fuse, by using the fusion layer, a plurality of pieces of initial feature data in one-to-one correspondence with the plurality of pieces of physiological time-series data to obtain fused feature data.

Exemplarily, the second prediction module is configured to perform, by using the fully connected layer, physiological state processing based on the fused feature data to obtain a prediction result.

Exemplarily, the adjustment module is configured to adjust a model parameter of the physiological state recognition model based on an error between the prediction result and the sample label data, to train the physiological state recognition model.

Exemplarily, the model parameter of the physiological state recognition model includes a weight of the neural network corresponding to each of the plurality of physiological time-series sample data.

Exemplarily, the second fusion module is further configured to: determine, for a plurality of neural networks in one-to-one correspondence with the plurality of pieces of physiological time-series data, a plurality of weights in one-to-one correspondence with the plurality of neural networks, in which the plurality of weights forms a weight vector; concatenate the plurality of pieces of initial feature data in one-to-one correspondence with the plurality of pieces of physiological time-series data to obtain a concatenated vector; and obtain the fused feature data by multiplying the concatenated vector by the weight vector.

Exemplarily, the second decomposition module is further configured to: perform the feature decomposition on each of the plurality of pieces of physiological time-series data to obtain trend subsequence data and periodic subsequence data, in which the trend subsequence data represents a changing trend of the physiological time-series data over time, and the periodic subsequence data represents a periodic variation characteristic of the physiological-time series data; and take at least one of the trend subsequence data or the periodic subsequence data as the subsequence data for each of the plurality of pieces of physiological time-series data.

Exemplarily, the physiological time-series data is obtained by adding the trend subsequence data and the periodic subsequence data or by multiplying the trend subsequence data by the periodic subsequence data.

Exemplarily, the second decomposition module is further configured to: perform the feature decomposition on each of the plurality of pieces of physiological time-series data to obtain trend subsequence data, periodic subsequence data, and error data; and remove the error data, and take trend subsequence data and periodic subsequence data as the subsequence data for each of the plurality of pieces of physiological time-series data.

Exemplarily, the second extraction module is further configured to input the subsequence data corresponding to each of the plurality of pieces of physiological time-series data into a neural network corresponding to each of the plurality of pieces of physiological time-series data for the feature extraction to obtain the initial feature data.

It can be understood that for the specific implementation process of the apparatus for training the physiological state recognition model, reference can be made to the implementation process of the method for training the physiological state recognition model mentioned above, and details thereof are omitted herein.

Embodiments of the present disclosure provide a computer-readable storage medium. The computer-readable storage medium has a computer program stored thereon. The computer program is configured to implement, when executed by a processor, the steps of the method in any one of the above-mentioned embodiments.

An embodiment of the present disclosure provides a computer program product. The computer program product includes instructions. The instructions, when executed by a processor of a computer device, causes the computer device to perform the steps of the method in any one of the above-mentioned embodiments.

Those skilled in the art can understand that the exemplary steps of the method described in combination with the embodiments disclosed herein can be implemented in electronic hardware, computer software or a combination thereof. In order to clearly illustrate interchangeability of the electronic hardware and the software, the exemplary composition and steps have been generally described in accordance with the functions in the above description. Whether these functions are executed by the electronic hardware or the software is dependent on particular use and design constraints of the technical solutions. Those skilled in the art can use different methods to implement the described functions for each specific application, but such an implementation should not be considered to be beyond the scope of the present disclosure.

So far, the technical solutions of the present disclosure have been described with reference to the preferred embodiments shown in the accompanying drawings. However, it is readily understood by those skilled in the art that the scope of the present disclosure is obviously not limited to these specific embodiments. Without departing from the principle of the present disclosure, those skilled in the art can make equivalent modifications or substitutions to the relevant technical features, and these modifications or substitutions all fall within the scope of the present disclosure.

## Claims

1. A method for training a human-factors intelligent physiological state recognition model, the method comprising:
obtaining a first physiological signal, a first state label of the first physiological signal, a second physiological signal corresponding to the first physiological signal, and a second state label of the second physiological signal, wherein the second physiological signal is a physiological signal generated based on the first physiological signal, the first physiological signal is a collected physiological signal, the first state label indicates a physiological state corresponding to the first physiological signal, and the second state label indicates a physiological state corresponding to the second physiological signal; and
training the physiological state recognition model based on the first physiological signal, the second physiological signal, the first state label, and the second state label.

2. The method according to claim 1, wherein said training the physiological state recognition model based on the first physiological signal, the second physiological signal, the first state label, and the second state label comprises:
generating a training sample of the physiological state recognition model based on the first physiological signal and the first state label of the first physiological signal, and generating another training sample of the physiological state recognition model based on the second physiological signal and the second state label of the second physiological signal; and
training the physiological state recognition model using the training samples.

3. The method according to claim 2, wherein said obtaining the second physiological signal corresponding to the first physiological signal comprises:
generating a feature vector of the second physiological signal based on the first physiological signal and the first state label of the first physiological signal; and
inputting the feature vector into a predetermined generator to obtain the second physiological signal outputted by the generator, the generator being configured to generate a physiological signal based on a feature vector.

4. The method according to claim 3, wherein said generating the feature vector of the second physiological signal based on the first physiological signal and the first state label of the first physiological signal comprises:
extracting a predetermined feature of the first physiological signal;
generating a feature vector of the first physiological signal based on the predetermined feature and the first state label of the first physiological signal; and
generating the feature vector of the second physiological signal based on the feature vector of the first physiological signal.

5. The method according to claim 4, wherein:
the physiological signal is an electrocardiogram signal; and
the predetermined feature is a heart rate variability, HRV, feature.

6. The method according to any one of claims 3 to 5, wherein the generator is trained by:
obtaining a fourth physiological signal and a fourth state label of the fourth physiological signal, the fourth physiological signal being a collected physiological signal;
generating a feature vector of a fifth physiological signal based on the fourth physiological signal and the fourth state label of the fourth physiological signal;
inputting the feature vector of the fifth physiological signal into the predetermined generator to obtain the fifth physiological signal outputted by the generator;
training a predetermined discriminator based on the fourth physiological signal and the fifth physiological signal;
obtaining a sixth physiological signal and a sixth state label of the sixth physiological signal, the sixth physiological signal being a collected physiological signal; and
performing adversarial training on the generator and the discriminator based on the sixth physiological signal and the sixth state label of the sixth physiological signal to obtain a trained generator.

7. The method according to claim 6, wherein said training the predetermined discriminator based on the fourth physiological signal and the fifth physiological signal comprises:
assigning a first source label to the fourth physiological signal and a second source label to the fifth physiological signal, the first source label indicating that the fourth physiological signal is the collected physiological signal, and the second source label indicating that the fifth physiological signal is a generated physiological signal; and
training the discriminator based on the fourth physiological signal, the first source label, the fifth physiological signal, and the second source label.

8. The method according to claim 6, wherein said performing the adversarial training on the generator and the discriminator based on the sixth physiological signal and the sixth state label of the sixth physiological signal to obtain the trained generator comprises:
generating a feature vector of a seventh physiological signal based on the sixth physiological signal and the sixth state label of the sixth physiological signal;
inputting the feature vector of the seventh physiological signal into the generator to obtain the seventh physiological signal outputted by the generator;
assigning a first source label to the sixth physiological signal and a second source label to the seventh physiological signal, the first source label indicating that the sixth physiological signal is the collected physiological signal, and the second source label indicating that the seventh physiological signal is a generated physiological signal;
generating a training sample of the discriminator based on the sixth physiological signal and the first source label, and generating another training sample of the discriminator based on the seventh physiological signal and the second source label;
inputting the training samples of the discriminator into the discriminator; and
adjusting a parameter of each of the generator and the discriminator based on a discrimination result outputted by the discriminator.

9. The method according to any one of claims 1 to 5, wherein the physiological state comprises a mental state, an emotional state, or a health state.

10. The method according to any one of claims 1 to 5, wherein the physiological state recognition model is implemented by a Bayesian neural network combined with a Transformer Encoder.

11. A method for physiological state recognition, the method comprising:
obtaining a physiological signal of a user;
inputting the physiological signal into a physiological state recognition model, the physiological state recognition model being configured to recognize a physiological state based on the physiological signal, and the physiological state recognition model being obtained by training with the method according to any one of claims 1 to 10; and
obtaining a physiological state recognition result outputted by the physiological state recognition model as a physiological state recognition result of the user.

12. The method according to claim 11, wherein:
the physiological signal comprises a plurality of pieces of physiological time-series data; and
said recognizing the physiological state based on the physiological signal comprises:
obtaining the plurality of pieces of physiological time-series data, each of the plurality of pieces of physiological time-series data being collected through a corresponding data collection channel;
performing feature decomposition on each of the plurality of pieces of physiological time-series data to obtain subsequence data for each of the plurality of pieces of physiological time-series data;
performing feature extraction on the subsequence data corresponding to each of the plurality of pieces of physiological time-series data to obtain initial feature data;
fusing a plurality of pieces of initial feature data in one-to-one correspondence with the plurality of pieces of physiological time-series data to obtain fused feature data; and
predicting the physiological state based on the fused feature data.

13. The method according to claim 12, wherein said performing the feature decomposition on each of the plurality of pieces of physiological time-series data to obtain the subsequence data for each of the plurality of pieces of physiological time-series data comprises:
performing the feature decomposition on each of the plurality of pieces of physiological time-series data to obtain trend subsequence data and periodic subsequence data, the trend subsequence data representing a changing trend of the physiological time-series data over time, and the periodic subsequence data representing a periodic variation characteristic of the physiological time-series data; and
taking at least one of the trend subsequence data or the periodic subsequence data as the subsequence data for each of the plurality of pieces of physiological time-series data.

14. The method according to claim 13, wherein the physiological time-series data is obtained by adding the trend subsequence data and the periodic subsequence data or by multiplying the trend subsequence data by the periodic subsequence data.

15. The method according to any one of claims 12 to 14, wherein said performing the feature extraction on the subsequence data corresponding to each of the plurality of pieces of physiological time-series data to obtain the initial feature data comprises:
inputting the subsequence data corresponding to each of the plurality of pieces of physiological time-series data into a neural network corresponding to each of the plurality of pieces of physiological time-series data for the feature extraction to obtain the initial feature data.

16. The method according to claim 15, wherein said fusing the plurality of pieces of initial feature data in one-to-one correspondence with the plurality of pieces of physiological time-series data to obtain the fused feature data comprises:
determining, for a plurality of neural networks in one-to-one correspondence with the plurality of pieces of physiological time-series data, a plurality of weights in one-to-one correspondence with the plurality of neural networks, the plurality of weights forming a weight vector;
concatenating the plurality of pieces of initial feature data in one-to-one correspondence with the plurality of pieces of physiological time-series data to obtain a concatenated vector; and
obtaining the fused feature data by multiplying the concatenated vector by the weight vector.

17. The method according to claim 11, wherein the physiological state recognition model comprises:
a hidden layer configured to perform feature decomposition on each of a plurality of pieces of physiological time-series data to obtain subsequence data for each of the plurality of pieces of physiological time-series data, each of the plurality of pieces of physiological time-series data being collected through a corresponding data collection channel;
a neural network layer configured to perform feature extraction on the subsequence data corresponding to each of the plurality of pieces of physiological time-series data to obtain initial feature data;
a fusion layer configured to fuse a plurality of pieces of initial feature data in one-to-one correspondence with the plurality of pieces of physiological time-series data to obtain fused feature data; and
a fully connected layer configured to predict the physiological state based on the fused feature data.

18. An apparatus for training a physiological state recognition model, the apparatus comprising:
an obtaining module configured to obtain a first physiological signal, a first state label of the first physiological signal, a second physiological signal corresponding to the first physiological signal, and a second state label of the second physiological signal, wherein the second physiological signal is a physiological signal generated based on the first physiological signal, the first physiological signal is a collected physiological signal, the first state label indicates a physiological state corresponding to the first physiological signal, and the second state label indicates a physiological state corresponding to the second physiological signal; and
a training module configured to train the physiological state recognition model based on the first physiological signal, the second physiological signal, the first state label, and the second state label.

19. An apparatus for physiological state recognition, the apparatus comprising:
an obtaining module configured to obtain a physiological signal of a target object; and
a recognition module configured to:
input the physiological signal into a predetermined physiological state recognition model, the physiological state recognition model being configured to recognize a physiological state based on the physiological signal, and the predetermined physiological state recognition model being obtained by training with the method according to any one of claims 1 to 9; and
obtain a physiological state recognition result outputted by the physiological state recognition model as a physiological state recognition result of the target object.

20. An electronic device, comprising:
a processor; and
a memory having one or more computer programs stored thereon, wherein the one or more computer programs comprise instructions, wherein the instructions, when executed by the processor, cause the electronic device to perform the method according to any one of claims 1 to 17.

21. A computer-readable storage medium, having a computer program stored therein, wherein the computer program, when executed by a computer, causes the computer to perform the method according to any one of claims 1 to 17.
